# EUROPEAN PATENT SPECIFICATION

(11) **EP 2 111 154 B1**
(45) Date of publication and mention of the grant of the patent: **05.10.2011**
(21) Application number: 08728188.7
(22) Date of filing: 24.01.2008
(51) Int. Cl.: A61B 5/0402, A61B 5/08

(54) **SYNCHRONIZING ULTRASOUND AND ECG DATA**
SYNCHRONISIERUNG VON ULTRASCHALL- UND EKG-DATEN
SYNCHRONISATION DE DONNÉES ULTRASONORES ET DE DONNÉES ECG

(30) Priority: 24.01.2007 US 886483 P
(43) Date of publication of application: 28.10.2009
(73) Proprietor: ImaCor Inc., Garden City, NY 11530 (US)
(72) Inventor: HASTINGS, Harold, M., Garden City, NY 11530 (US)
(74) Representative: Howard, Paul Nicholas
(86) International application number: PCT/US2008/051886
(87) International publication number: WO 2008/091990

(56) References cited:
- EP-A- 1 779 787
- US-A1- 2006 058 662

## Description

### BACKGROUND

The operation of the heart of a patient can be monitored in vivo using a variety of approaches. One commonly used approach for monitoring the operation of the heart is an electrocardiogram (ECG or EKG) which is a graphical representation of the electrical activity of the heart over time. Another commonly used approach for monitoring the operation of the heart is the echocardiogram, which is typically used to generate a two dimensional moving video image of the heart in real time, while the heart is beating. Each of these approaches provides a different set of information about the operation of the heart, in real time. US 2006 058 662 discloses the synchronization of ultrasound and Ecg Data by inserting a common time stamp in both image streams

### SUMMARY

The present invention provides a method as recited in the claims. ECG data and ultrasound data are synchronized by adding a marker to both sets of data, then detecting the position of the markers in the data, and using the detected positions to align the two sets of data in time. This enables an operator to visualize which frame of ultrasound data corresponds in time to which portion of the ECG waveform.

### BRIEF DESCRIPTION OF THE DRAWINGS

FIG. 1 is a graph of a set of waveforms that are introduced into the ECG and ultrasound systems, to form the markers in the respective data.

FIG. 2 is a schematic diagram of a circuit that is suitable for generating the markers for the ECG and ultrasound systems.

FIG. 3 is a schematic diagram of an alternative circuit that is suitable for generating the markers for the ECG and ultrasound systems.

### DETAILED DESCRIPTION OF THE PREFERRED EMBODIMENTS

The inventor has recognized that significant advantages can be obtained b7y synchronizing ECG data and ultrasound data so that the operator can visualize which frame of ultrasound data corresponds in time to which portion of the ECG waveform, and the present invention relates to synchronizing or registering ECG and ultrasound data. While the primary intended application is in the field of cardiac ultrasound, it can be used in numerous other applications as well.

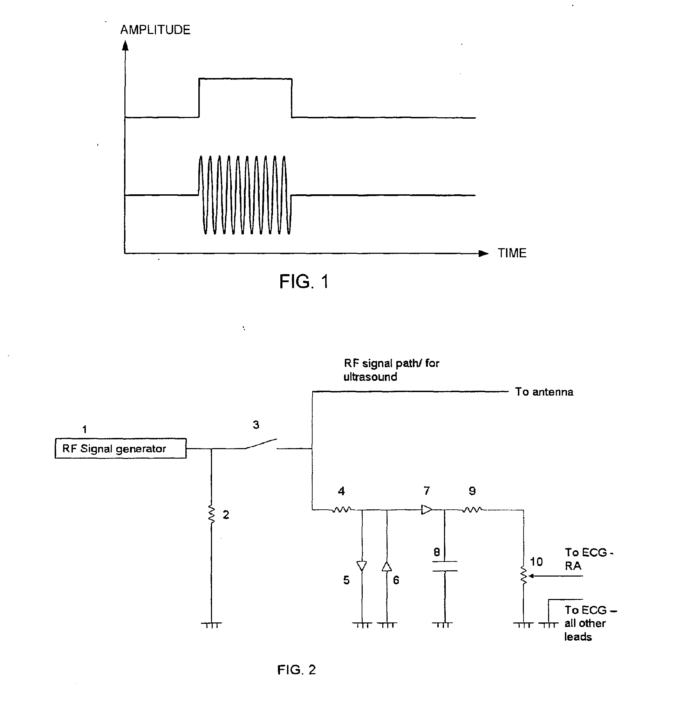

In one preferred implementation, synchronization is provided by simultaneously generating (a) a first signal that can be detected by an ECG machine; and (b) a second signal that can be detected by an ultrasound machine. One suitable set of such signals is depicted in FIG. 1, in which the top trace can be detected by an ECG machine and the bottom trace can be detected by an ultrasound machine.

For the signal that is detectable by the ECG machine, a short, positive-going pulse of current from the baseline for the ECG signal is suitable. For the signal that is detectable by the ultrasound machine, a short burst of ultrasound RF is suitable, with the RF carrier preferably close to the operating frequency of the transducer (e.g., 6 MHz). Preferably, the signal should be at least as long as the frame-to-frame interval of the ultrasound system. For example, a 20 mS pulse would be suitable for a system that is imaging at 50 frames per second. Preferably, the rise and fall times of the pulse should be much shorter than the frame-to-frame interval (e.g., less than 1 mS).

The burst of RF is synchronized with the pulse that is applied to the ECG machine, e.g., as shown in FIG. 1. Note that while FIG. 1 schematically depicts the lower waveform with ten cycles of the wave, many more cycles of the wave will be present in the waveforms that are actually used. For example, with a 6 MHz RF signal, there would be 120,000 cycles in a 20 mS burst.

The required synchronized pulses can be generated with the circuit depicted in FIG. 2. A commercially available function generator (e.g., BK Precision 4017A) may be used as the signal generator 1, in which case it should preferably be set to match the center frequency of the ultrasound transducer that is being used. For example, if the center frequency of the ultrasound transducer is 6 MHz, the signal generator 1 should be set to generate a 6 MHz sine wave. Alternatively, a sine wave oscillator may be custom designed to operate at the desired frequency, in which case the entire circuit can be battery operated, and optionally electrically isolated. Resistor 2 is a load resistor that preferably matches the output impedance of the signal generator (e.g., 50 Ω).

Switch 3 may be a manually operated switch that, when actuated, passes the output of the signal generator 1 to the rest of the circuit. Alternatively, it may be an electronic switch that is closed for a predetermined period of time (e.g., between about 10 and about 50 mS, and preferably about 10 mS), under control of a suitable circuit (e.g., a one-shot).

Resistor 4 (e.g., 100 Ω) and back to back diodes 5 and 6 form a protective circuit to limit the voltage that is applied to the ECG system. Diode 7 half-wave rectifies the AC, and capacitor 8 (e.g., 1000 pF) captures the peaks. The capacitor 8 is discharged through resistor 9 (e.g., 3 kΩ) and the track of potentiometer 10 (e.g., 10 Ω), and the output voltage is set to match the ECG system by adjusting the position of the wiper of the potentiometer 10. Taken together, Diode 7, capacitor 8, resistor 9, and potentiometer 10 operate as an envelope detector.

To adjust the circuit for operation, the ultrasound box is set up for imaging, and the antenna is placed near the ultrasound probe or near the connector to the ultrasound box.
(A physical connection to the ultrasound box is not required to couple the signal into the ultrasound box.) Closing switch 3 sends RF to the antenna, and the signal generator output level and/or antenna position is adjusted until the ultrasound image appears noticeable whiter than it was when the switch 3 was open. This increase in whiteness serves as a marker or artifact in the ultrasound image. Next, the output from the envelope detector is connected to the ECG machine. One suitable way to make this connection is by connecting the output from the envelope detector to the RA input of the ECG machine and connecting the ground to the other leads of the ECG machine, but other lead connection arrangements may also be used, as will be apparent to persons skilled in the relevant arts. Pulses are then generated by closing and opening the switch 3, and adjusting potentiometer 10 until a satisfactory signal appears on the ECG, in the form of the leading edge of a positive going pulse, analogous to the upstroke of an R-wave. This pulse serves as a marker or artifact in the electrocardiogram.

After the adjustment, the ultrasound system and the ECG system are operated simultaneously to capture images and an electrocardiogram of the subject. While this is happening, pulses may be generated by closing and opening S3, and the ultrasound images and the electrocardiogram are recorded. Afterwards, the ultrasound image is played back frame by frame, and the appearance of the marker in the ultrasound images is compared to the appearance of the marker in the electrocardiogram. A timing relationship between the ultrasound image and the electrocardiogram can then be determined. Alternatively, a pulse train generator may be used to generate pulses at regular intervals, and a timing relationship between the ultrasound image and the electrocardiogram may then be determined using the pulse repetition rate.

Once the timing relationship between the two systems has been established, it can be calculated for subsequent times by tracking the amount of time elapsed in both systems.
Since only one frame of an ultrasound image is typically displayed at any given instant, and an electrocardiogram displays a number of seconds of data all at once, one suitable user interface for indicating the timing relationship between the ultrasound images and the electrocardiogram is to colorize the spot on the electrocardiogram trace that corresponds to the frame of ultrasound that is being displayed at any given instant. When the ultrasound image is played back, the colorized spot would then move along the electrocardiogram trace. Alternatively, if the current time corresponds to a fixed position on the electrocardiogram display screen, the fixed position could be marked on the screen using a vertical line, and the ultrasound frame that corresponds to whatever portion of the electrocardiogram is at that fixed position at any given instant could be displayed. A wide variety of alternative user interfaces for displaying both sets of information and indicating the timing relationship between them can be readily envisioned.

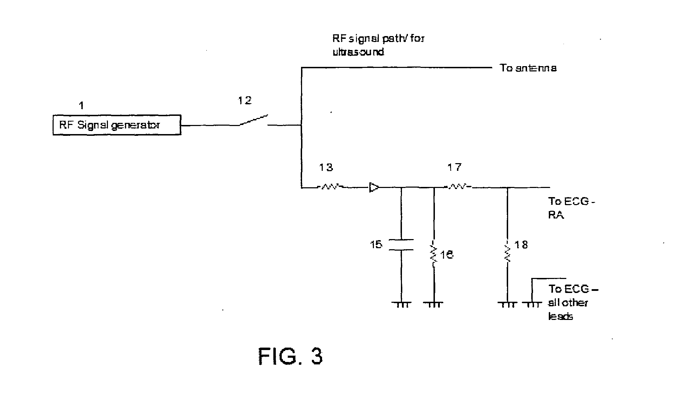

FIG. 3 is a schematic diagram of an alternative circuit for generating the markers in the ultrasound and the electrocardiogram. It is similar to the circuit shown in FIG. 2, except that the protection diodes 5 and 6 are omitted, and the combination of the resistor 9 and the potentiometer 10 (which did double-duty of both discharging the capacitor and dividing down the voltage) are replaced by a fixed resistor 16 for discharging the capacitor, and a pair of resistors 17 and 18 for dividing down the voltage. A suitable set of component values for this embodiment is: 200 Ω for R13, 10 kΩ for R16, 220 kΩ for R17, 200 Ω for R18, and 1000 pF for C15.

Synchronizing the ultrasound image with the ECG can be especially important in cardiac resynchronization therapy (CRT). In CRT one is interested the use of bi-ventricular pacemakers to overcome problems in the timing of cardiac wall movement - dyssynchrony.
In particular, one would like to know that contraction is appropriately synchronous throughout the left ventricle - except for a smooth gradient from apex to base. Ultrasound data accurately timed with respect to the ECG and in particular the R-wave, for example, in the form of CINE loops, could be used to assess the appropriateness of the placement of pacemaker leads and the timing of pacemaker impulses.

## Claims

1. A method of synchronizing an electrocardiogram with an ultrasound images the method comprising the steps of:
obtaining an electrocardiogram during at least one beat of a subject's heart using an Ecg system;
obtaining an ultrasound image of the subject using an ultrasound system, wherein the ultrasound image includes enough frames to depict at least one beat of the subject's heart; and :
coupling, into the Ecg system, a first signal that introduces a marker into the electrocardiogram;
coupling, into the ultrasound system, a second signal that introduces a marker into the ultrasound image, wherein a known time relationship exists between the first signal and the second signal,
**characterized in that** the first signal comprises a rectangular pulse and the second signal comprises a burst of RF at a frequency that is detectable by the ultrasound system, further comprising the steps of:
identifying at least one portion of the electrocardiogram which the first marker appears;
identifying at least one frame of the ultrasound image that contains the second marker; and
aligning the electrocardiogram and the ultrasound image in time based on (a) the identified at least one portion of the electrocardiogram, (b) the identified at least one frame of the ultrasound image, and (c) the known time relationship.

2. The method of claim 1, wherein the first signal and the second signal are substantially simultaneous.

3. The method of any one of claims 1 to 2, wherein the first signal comprises a rectangular pulse and the second signal comprises a burst of RF with a center frequency between about 4 and about 8 MHz, and the duration of both the first signal and the second signal is between about 10 and about 50 mS.

4. The method of any one of claims 1 to 2, wherein the first signal comprises a rectangular pulse and the second signal comprises a burst of RF with a center frequency between about 4 and about 8 MHz, and the duration of both the first signal and the second signal is about 20 mS.

5. The method of any of claims 1 to 4, wherein the first signal comprises a plurality of rectangular pulses spaced at regular intervals, and the second signal comprises a plurality of bursts of RF at a frequency that is detectable by the ultrasound system, spaced at the regular intervals.

## Patentansprüche

1. Verfahren zum Synchronisieren eines Elektrokardiogramms mit einem Ultraschallbild, wobei das Verfahren die folgenden Schritte umfasst:
Erhalten eines Elektrokardiogramms während mindestens eines Herzschlags des Herzens eines Subjekts unter Verwendung eines EKG-Systems;
Erhalten eines Ultraschallbildes des Subjekts unter Verwendung eines Ultraschallsystems, wobei das Ultraschallbild genug Einzelbilder zum Abbilden mindestens eines Herzschlags des Herzens des Subjekts umfasst; und
Koppeln eines ersten Signals, das eine Markierung in das Elektrokardiogramm einführt, in das EKG-System;
Koppeln eines zweiten Signals, das eine Markierung in das Ultraschallbild einführt, in das Ultraschallsystem, wobei zwischen dem ersten Signal und dem zweiten Signal eine bekannte Zeitbeziehung existiert,
**dadurch gekennzeichnet, dass** das erste Signal einen Rechteckimpuls umfasst und das zweite Signal einen Burst von HF mit einer Frequenz umfasst, die durch das Ultraschallsystem detektierbar ist, ferner mit den folgenden Schritten:
Identifizieren mindestens eines Teils des Elektrokardiogramms, indem die erste Markierung erscheint;
Identifizieren mindestens eines Einzelbildes des Ultraschallbildes, das die zweite Markierung enthält;
und
zeitliches Synchronisieren des Elektrokardiogramms und des Ultraschallbildes auf der Basis (a) des identifizierten mindestens einen Teils des Elektrokardiogramms, (b) des identifizierten mindestens einen Einzelbildes des Ultraschallbildes und (c) der bekannten Zeitbeziehung.

2. Verfahren nach Anspruch 1, wobei das erste Signal und das zweite Signal im Wesentlichen simultan sind.

3. Verfahren nach einem der Ansprüche 1 bis 2, wobei das erste Signal einen Rechteckimpuls umfasst und das zweite Signal einen Burst von HF mit einer Mittenfrequenz zwischen etwa 4 und etwa 8 MHz umfasst und die Dauer sowohl des ersten als auch des zweiten Signals zwischen etwa 10 und etwa 50 ms beträgt.

4. Verfahren nach einem der Ansprüche 1 bis 2, wobei das erste Signal einen Rechteckimpuls umfasst und das zweite Signal einen Burst von HF mit einer Mittenfrequenz zwischen etwa 4 und etwa 8 MHz umfasst und die Dauer sowohl des ersten Signals als auch des zweiten Signals etwa 20 ms beträgt.

5. Verfahren nach einem der Ansprüche 1 bis 4, wobei das erste Signal mehrere in regelmäßigen Intervallen beabstandete Rechteckimpulse umfasst und das zweite Signal mehrere Bursts von HF mit einer Frequenz umfasst, die durch das Ultraschallsystem detektierbar ist, die in den regelmäßigen Intervallen beabstandet sind.

## Revendications

1. Procédé de synchronisation d'un électrocardiogramme et d'une image par ultrasons, le procédé comprenant les étapes consistant à :
obtenir un électrocardiogramme au cours d'au moins un battement du coeur d'un patient au moyen d'un système ECG ;
obtenir une image par ultrasons du patient au moyen d'un système à ultrasons, l'image par ultrasons comportant suffisamment d'images isolées pour représenter au moins un battement du coeur du patient ; et
coupler, dans le système ECG, un premier signal qui introduit un marqueur dans l'électrocardiogramme ;
coupler, dans le système à ultrasons, un deuxième signal qui introduit un marqueur dans l'image par ultrasons, une relation temporelle connue existant entre le premier signal et le deuxième signal,
le procédé étant **caractérisé en ce que** le premier signal comprend une impulsion rectangulaire et le deuxième signal comprend une salve radiofréquence à une fréquence détectable par le système à ultrasons, et **en ce qu'**il comprend en outre les étapes consistant à :
identifier au moins une partie de l'électrocardiogramme dans laquelle le premier marqueur apparaît ;
identifier au moins une image isolée de l'image par ultrasons qui contient le deuxième marqueur ; et
aligner temporellement l'électrocardiogramme et l'image par ultrasons compte tenu (a) de ladite au moins une partie identifiée de l'électrocardiogramme, (b) de ladite au moins une image isolée identifiée de l'image par ultrasons, et (c) de la relation temporelle connue.

2. Procédé selon la revendication 1, le premier signal et le deuxième signal étant sensiblement simultanés.

3. Procédé selon l'une quelconque des revendications 1 à 2, le premier signal comprenant une impulsion rectangulaire et le deuxième signal comprenant une salve radiofréquence dont la fréquence centrale est comprise entre environ 4 et environ 8 MHz, et la durée du premier signal ainsi que du deuxième signal étant comprise entre environ 10 et environ 50 ms.

4. Procédé selon l'une quelconque des revendications 1 à 2, le premier signal comprenant une impulsion rectangulaire et le deuxième signal comprenant une salve radiofréquence dont la fréquence centrale est comprise entre environ 4 et environ 8 MHz, et la durée du premier signal ainsi que du deuxième signal étant d'environ 20 ms.

5. Procédé selon l'une quelconque des revendications 1 à 4, le premier signal comprenant une pluralité d'impulsions rectangulaires séparées par des intervalles réguliers et le deuxième signal comprenant une pluralité de salves radiofréquences à une fréquence détectable par le système à ultrasons, séparées par les intervalles réguliers.
